## Europäisches Patentamt
## European Patent Office
## Office européen des brevets

(19)

(11) Numéro de publication: **0 009 423**
**B1**

(12) **FASCICULE DE BREVET EUROPEEN**

(45) Date de publication du fascicule du brevet: 05.05.82

(21) Numéro de dépôt: 79400565.2

(22) Date de dépôt: 10.08.79

(51) Int. Cl.³: **A 61 K 9/02, C 08 J 7/18, A 61 F 5/47, A 61 M 31/00**

(54) **Substrat hydrophobe apte à libérer une substance chimique, procédé pour sa préparation et son application comme dispositif intra-utérin.**

(30) Priorité: 25.08.78 FR 7824678

(43) Date de publication de la demande:
02.04.80 Bulletin 80/7

(45) Mention de la délivrance du brevet:
05.05.82 Bulletin 82/18

(84) Etats contractants désignés:
BE CH DE GB IT NL SE

(56) Documents cités:
FR - A - 2 250 520
FR - A - 2 250 793
FR - A - 2 278 317
FR - A - 2 367 087
FR - E - 2 348 238

CHEMICAL ABSTRACTS, vol. 87, no. 24 12-12-1977, page 309, ref. 189410u
Columbus, Ohio, US
G. GAUSSENS et al.: "Synthesis of copolymers suitable for the storage of slow release of reactants. Cases of copper salts for intra-uterine devices"

(73) Titulaire: **COMMISSARIAT A L'ENERGIE ATOMIQUE Etablissement de Caractère Scientifique Technique et Industriel**
B.P. 510
F-75752 Paris Cedex 15 (FR)

(72) Inventeur: **Berthet, Jeanne**
**1, Allée du Roussillon**
**F-78140 Velizy Villacoublay (FR)**
Inventeur: **Gaussens, Gilbert**
**11, Rue Jean Brunet**
**F-92190 Meudon (FR)**

(74) Mandataire: **Mongrédien, André et al,**
**c/o Brevatome 25, rue de Ponthieu**
**F-75008 Paris (FR)**

Courier Press, Leamington Spa, England.

# 0 009 423

Substrat hydrophobe apte à libérer une substance chimique, procédé pour sa préparation
et son application comme dispositif intra-utérin.

La présente invention a pour objet un substrat hydrophobe apte à libérer une substance chimique et un procédé de fabrication d'un tel substrat.

On sait que des substrats présentant la propriété de pouvoir libérer une substance chimique à une vitesse contrôlée, pendant une durée prolongée, présentent un grand intérêt dans de nombreux domaines, en particulier dans le domaine médical.

En effet, de tels substrats peuvent être utilisés pour l'introduction dans le corps humain d'une substance active telle qu'un médicament et ils trouvent en particulier une application à la réalisation de dispositifs intra-utérins susceptibles de libérer dans l'utérus, pendant une durée prolongée, une dose sensiblement constante d'une substance active telle qu'un agent anticonceptionnel.

Les procédés connus actuellement pour préparer des substrats de ce type font appel à une première étape de formation d'inclusions hydrophiles ou hydrophobes dans un substrat hydrophobe et à une deuxième étape qui consiste à absorber une substance chimique dans les inclusions hydrophiles ou hydrophobes du substrat par immersion du substrat dans une solution de la substance.

Ces procédés sont en particulier décrits dans les brevets français 2.250793 et 2.250.520 qui illustrent la réalisation d'un substrat hydrophobe contenant des inclusions hydrophiles dans lesquelles est stockée une substance chimique soluble dans un solvant polaire, et dans le brevet français 2.367.087 qui illustre la réalisation d'un substrat hydrophobe comportant des inclusions hydrophobes dans lesquelles sont stockées des substances hydrophobes ou solubles dans un solvant hydrophobe.

Dans ces substrats connus, la substance est stockée dans les inclusions par absorption et de ce fait la quantité de substance chimique stockée est limitée par le teneur en inclusions hydrophiles ou hydrophobes du substrat, par la capacité d'absorption du polymère constituant ces inclusions et par la durée de l'étape d'absorption. De plus, pour obtenir cette absorption de la substance chimique dans les inclusions, il est nécessaire de réaliser l'étape d'immersion du substrat greffé dans la solution de la substance pendant une durée relativement longue afin d'atteindre le maximum d'absorption.

La présente invention a précisément pour objet un substrat hydrophobe apte à libérer une substance chimique, qui présente justement l'avantage de pouvoir renfermer une quantité plus importante de substance chimique que les substrats actuellement connus et qui par ailleurs peut être réalisé par un procédé ne nécessitant pas la mise en oeuvre d'une étape de stockage de la substance à partir d'une solution.

Selon l'invention, le substrat hydrophobe apte à libérer au moins une substance chimique, comprend une matrice de polymère hydrophobe contenant une substance chimique et comportant des inclusions de composés polymérisés, hydrophiles ou hydrophobes, différents du polymère hydrophobe, greffés sur ce polymère hydrophobe, et il se caractérise en ce que ladite substance est stockée dans la matrice à l'état solide en dehors des inclusions de composés polymérisés.

Le substrat hydrophobe tel que caractérisé ci-dessus tire avantageusement profit de se structure particulière qui lui permet de libérer, dans certaines conditions, la substance chimique qu'il contient, en raison de la présence du réseau stable d'inclusions de composés polymérisés, qui constitue dans la matrice de polymère hydrophobe une voie d'accès vers les inclusions de la substance chimique.

Selon un premier mode de réalisation de l'invention, les inclusions de composés polymérisés sont des inclusions de composés hydrophiles, par exemple des inclusions de polymère d'un monomère hydrophile choisi dans le groupe comprenant l'acrylamide, l'acrylate d'éthylène glycol, le méthylol-acrylamide, la diacétone acrylamide, l'acide maléique, l'acide acrylique, l'acide fumarique, l'acide itaconique, l'acrylate de propylène glycol, le méthacrylate d'éthylène glycol, la méthacrylamide, l'acide méthacrylique et le méthacrylate de propylène glycol.

Ce mode de réalisation se révèle particulièrement avantageux pour libérer des substances chimiques dans une solution polaire étant donné que les inclusions hydrophiles possèdent la propriété d'absorber les solutions polaires.

En effet, lorsqu'on immerge un tel substrat dans une solution polaire, cette dernière peut pénétrer à l'intérieur de la matrice de polymère hydrophobe en suivant le trajet des inclusions hydrophiles pour venir en contact avec les inclusions de substance chimique, puis réagir avec cette substance ou la solubiliser et enfin migrer à l'extérieur du substrat en suivant également le trajet des inclusions hydrophiles pour libérer la substance à l'extérieur du substrat.

Dans ce premier mode de réalisation de l'invention, la substance chimique est avantageusement un métal ou un sel métallique tel que le cuivre ou l'acétate de cuivre.

Selon un second mode de réalisation de l'invention, les inclusions de composés polymérisés sont des inclusions de composés hydrophobes, par exemple des inclusions de polymère d'un monomère hydrophobe choisi dans le groupe comprenant l'acrylate d'éthyle, l'acrylate de butyle, l'acrylate d'isobutyle, l'acrylate d'hexyle, l'acrylate d'heptyle, le méthacrylate d'éthyle, le méthacrylate de butyle, le méthacrylate d'isobutyle, le méthacrylate d'hexyle, le méthacrylate d'heptyle et l'acrylonitrile.

Ce deuxième mode de réalisation de l'invention se révèle particulièrement avantageux pour libérer une substance dans un milieu susceptible d'être absorbé par les inclusions hydrophobes.

Dans ce cas, lorsqu'on met en contact un tel substrat avec un milieu approprié, ce dernier peut

2

pénétrer à l'intérieur de la matrice de polymère hydrophobe en suivant le trajet des inclusions hydrophobes pour venir en contact avec les inclusions de substance chimique, puis réagir avec cette substance ou la solubiliser, et enfin migrer à l'extérieur du substrat en suivant également le trajet des inclusions hydrophobes pour libérer la substance à l'extérieur du substrat.

Dans les deux modes de réalisation de l'invention, le polymère hydrophobe de la matrice est avantageusement choisi dans le groupe comprenant les acétates de polyvinyle, les polyéthylènes, les polypropylènes, les polyamides, le polytéréphtalate d'éthylène glycol, les chlorures de polyvinyle, les chlorures de polyformaldéhyde, les polycarbonates, les copolymères d'éthylène, les polyéthers, les polyuréthanes, les polyacrylonitriles et les copolymères d'éthylène et d'acétate de vinyle.

La présente invention a également pour objet un procédé de préparation du substrat hydrophobe apte à libérer une substance chimique, décrit ci-dessus.

Ce procédé consiste:

a) à former dans une poudre de polymère hydrophobe des inclusions de composés polymérisés en greffant sur la poudre de polymère hydrophobe un monomère hydrophile ou hydrophobe différent dudit polymère hydrophobe.

b) à mélanger de façon homogène la poudre greffée ainsi obtenue avec une poudre de ladite substance, et

c) à mettre le mélange sous la forme d'un substrat en le soumettant à un formage à chaud.

Le procédé tel que caractérisé ci-dessus tire avantageusement profit du fait qu'en créant par greffage des inclusions de composés polymérisés dans une poudre de polymère hydrophobe et en soumettant ensuite à une opération de formage à chaud un mélange de la poudre greffée et d'une poudre de la substance chimique, on obtient un substrat comportant une matrice de polymère hydrophobe dans laquelle sont réparties des inclusions de la substance chimique et des inclusions de composés polymérisés qui, grâce à la présence des liaisons covalentes formées lors du greffage, forment à l'intérieur de la matrice un réseau stable en contact avec les inclusions de la substance chimique.

Selon l'invention, le greffage du monomère sur la poudre de polymère hydrophobe peut être réalisé par tout procédé connu, par exemple, par voie chimique ou par irradiation au moyen de rayonnements ionisants tels que les rayonnements X, gamma, ultraviolets ou par un faisceau d'électrons.

Selon l'invention, on réalise de préférence le greffage dudit monomère sur ladite poudre de polymère hydrophobe en soumettant à une irradiation au moyen de rayonnements ionisants ladite poudre de polymère hydrophobe immergée dans une solution dudit monomère contenant un inhibiteur de polymérisation.

Dans ce mode de réalisation, on contrôle le taux de greffage dudit monomère sur le polymère hydrophobe en réglant la température et la concentration en monomère de la solution, le débit de dose d'irradiation et la durée d'irradiation.

Le taux de greffage est choisi en fonction de la nature du polymère hydrophobe et du monomère utilisé pour le greffage, de façon telle que le substrat obtenu comporte une quantité suffisante d'inclusions de composés polymérisés, tout en présentant, par ailleurs, les propriétés mécaniques intéressantes de la matrice hydrophobe, par exemple la souplesse mécanique dans le cas de la réalisation de substrats destinés à être utilisés commu dispositifs intra-utérins.

Après greffage, on mélange intimement la poudre greffée obtenue avec une poudre de la substance chimique à emmagasiner dans le substrat. Cette opération peut être réalisée dans un mélangeur à poudres de type classique, par exemple dans un mélangeur Moritz.

De préférence, pour la réalisation du mélange, on utilise une poudre de substance chimique présentant une granulométrie inférieure à celle de la poudre de polymère hydrophobe greffé, afin d'obtenir une dispersion fine et homogène de la substance chimique dans la matrice de polymère hydrophobe.

Le mélange obtenu est ensuite mis sous la forme d'un substrat par formage à chaud par exemple par extrusion, par injection ou par compression, à une température inférieure à la température de fusion de la substance chimique.

Dans certains cas, après formage à chaud, on soumet le substrat obtenu à une réticulation au moyen de rayonnements ionisants.

En effet, si la vitesse de migration de la substance chimique au travers des inclusions de composés polymérisés est trop importante, elle peut être ajustée en procédant à une réticulation sous rayonnement des inclusions après l'opération de formage. Ceci bien sûr à la condition que la substance chimique supporte cette irradiation.

Selon l'invention, le polymère hydrophobe est avantageusement choisi dans le groupe comprenant les acétates de polyvinyle, les polyéthylènes, les polypropylènes, les polyamides, le polytéréphtalate d'éthylène glycol, les chlorures de polyvinyle, les chlorures de polyformaldéhyde, les polycarbonates, les copolymères d'éthylènes, les polyéthers, les polyuréthanes, les polyacrylonitriles et les copolymères d'éthylène et d'acétate de vinyle.

Selon le procédé de l'invention, le monomère utilisé pour le greffage est un monomère hydrophile tel que l'acrylamide, l'acrylate d'éthylène glycol, le méthylol acrylamide, la diacétone acrylamide, l'acide maléique, l'acide acrylique, l'acide fumarique, l'acide itaconique, l'acrylate de propylène glycol, le

**0 009 423**

méthacrylate d'éthylène glycol, la méthacrylamide, l'acide méthacrylique et le méthacrylate de propylène glycol, ou un monomère hydrophobe tel que l'acrylate d'éthyle, l'acrylate de butyle, l'acrylate d'isobutyle, l'acrylate d'hexyle, l'acrylate d'heptyle le méthacrylate d'éthyle, le méthacrylate de butyle, le méthacrylate d'isobutyle, le méthacrylate d'hexyle, le méthacrylate d'heptyle et l'acrylonitrile.

Selon l'invention, lorsque le monomère utilisé pour le greffage est un monomère hydrophile, la substance chimique est, de préférence, une substance soluble en solution polaire ou une substance apte à réagir avec une solution polaire.

A titre d'exemple de substances susceptibles d'être utilisées, on peut citer en particulier, les métaux tels que le cuivre et les sels métalliques solubles dans l'eau ou dans une solution polaire, tels que l'acétate de cuivre. On peut également utiliser des substances actives, en particulier des médicaments, à condition bien entendu qu'ils soient solides à la température ambiante et restent stables à la température utilisée pour le formage du substrat.

On précise que selon le procédé de l'invention, le polymère hydrophobe et le monomère utilisés pour le greffage sont choisis en fonction de la nature et des propriétés de la substance chimique à emmagasiner dans le substrat de façon à conférer au substrat obtenu la propriété de pouvoir libérer la substance dans un milieu approprié, par exemple dans un gaz, une solution polaire ou un milieu liquide non polaire.

De plus, le polymère hydrophobe est choisi en fonction de ses propriétés mécaniques et chimiques, compte tenu de l'utilisation ultérieure du substrat obtenu. Ainsi, dans le cas d'une utilisation du substrat comme dispositif intra-utérin, on choisit un polymère hydrophobe présentant une grande souplesse mécanique.

L'invention sera mieux comprise à la lecture des exemples suivants sonnés bien entendu à titre illustratif et non limitatif et se référant au dessin annexé sur lequel:

— la fig. 1 est une représentation graphique, en fonction du temps, de la quantité de cuivre libérée par jour par le substrat obtenu dans l'exemple 1, lorsqu'on met en contact ce dernier avec une solution dont le pH est ajusté à 6,5, à une température de 37°C;

— la fig. 2 est une représentation graphique, en fonction du temps, de la quantité de cuivre libérée par jour par le substrat obtenu dans l'exemple 1, lorsqu'on met en contact ce dernier avec une solution dont le pH est ajusté à 5, à une température de 37°C;

— la fig. 3 est une représentation graphique, en fonction du temps, de la quantité de cuivre libérée par jour par le substrat obtenu dans l'exemple 2, lorsqu'on met et contact ce dernier avec une solution dont le pH a été ajusté à 6,5, à une température de 37°C;

— la fig. 4 est une représentation graphique, en fonction du temps, de la quantité de cuivre libérée par jour par le substrat obtenu dans l'exemple 2, lorsqu'on met en contact ce dernier, à une température de 37°C, avec une solution aqueuse dont le pH a été ajusté a 5;

— la fig. 5 est une représentation schématique d'un dispositif intra-utérin;

— la fig. 6 est une représentation graphique, en fonction du temps, de la quantité de cuivre libérée par jour par le dispositif intra-utérin de taille 0 obtenu dans l'exemple 3, lorsqu'on met en contact ce dispositif, à une température de 37°C, avec une solution dont le pH a été ajusté à 6,5;

— la fig. 7 est une représentation graphique, en fonction du temps, de la quantité de cuivre libérée par jour par le dispositif intra-utérin de taille 0, obtenu dans l'exemple 3, lorsqu'on met en contact ce dispositif, à une température de 37°C, avec une solution dont le pH a été ajusté à 5;

— la fig. 8 est une représentation graphique, en fonction du temps, de la quantité de cuivre libérée par jour par le dispositif intra-utérin de taille 1 obtenu dans l'exemple 3, lorsqu'on met en contact ce dispositif, à une température de 37°C, avec une solution dont le pH a été ajusté à 6,5;

— la fig. 9 est une représentation graphique, en fonction du temps, de la quantité de cuivre libérée par un dispositif intra-utérin de taille 1, obtenu dans l'exemple 3, lorsqu'on met en contact ce dernier, à une température de 37°C, avec une solution dont le pH a été ajusté à 5; et

— la fig. 10 est une représentation graphique, en fonction du temps, de la quantité de cuivre libérée par jour par le substrat obtenu dans l'exemple 4, lorsqu'on met en contact ce dernier avec une solution dont le pH a été ajusté à 5 à une température de 37°C.

Exemple 1

On greffe une poudre de copolymère d'éthylène et d'acétate de vinyle (EVA) à 33 % en poids d'acétate de vinyle, en utilisant la solution de greffage suivante:

— acide acrylique: 62,5 ml;
— $H_2O$ permutée: 500 ml;
— sel de Mohr: 11 g (inhibiteur de polymérisation).

Pour réaliser le greffage, on soumet 125 g de la poudre de copolymère EVA d'une granulométrie moyenne de 1 mm, immergée dans la solution de greffage et sous agitation, à une irradiation sous rayonnements gamma, au moyen d'une source de cobalt 60, sous un débit de dose de 0,72 Mrad/h, pendant une durée de 1h30, sous atmosphère d'azote, et à la température de 20°C.

Après irradiation, la poudre greffée est lavée puis séchée dans une étuve sous vide. On obtient

4

ainsi une poudre de copolymère greffé à l'acide acrylique, dont le taux de greffage défini par la formule:

$$\frac{P - Po}{Po} \times 100$$

dans laquelle P représente le poids de la poudre après greffage et Po le poids de la poudre avant greffage, est de 36 % en poids par rapport au poids de copolymère (EVA).

On mélange ensuite la poudre greffée avec 12,5 % en poids par rapport au poids de poudre greffée, de poudre de cuivre, d'une granulométrie moyenne de 50 microns, à une température de 140 à 150°C, dans un malaxeur, puis on introduit le mélange dans un moule de 1 mm d'épaisseur, dont les plateaux sont maintenus à une température de 170°C.

On vérifie ensuite les propriétés de désorption du substrat ainsi préparé, après avoir découpé dans la plaquette obtenue un morceau de plaquette d'une surface de 600 mm², c'est-à-dire d'une surface qui correspond sensiblement à la surface d'un dispositif intra-utérin de type classique. Dans ce but, on immerge le morceau de plaquette dans un litre de solution isotonique (9 g de NaCl/1) maintenue à une température de 37°C et dont le pH est ajusté à 5 ou à 6,5 per addition d'acide lactique pour réaliser une désorption du cuivre dans la solution. On change la solution à intervalles réguliers et on détermine la quantité de cuivre libérée dans chacune des solutions prélevées par spectrophotomètrie du complexe dithizonate de cuivre.

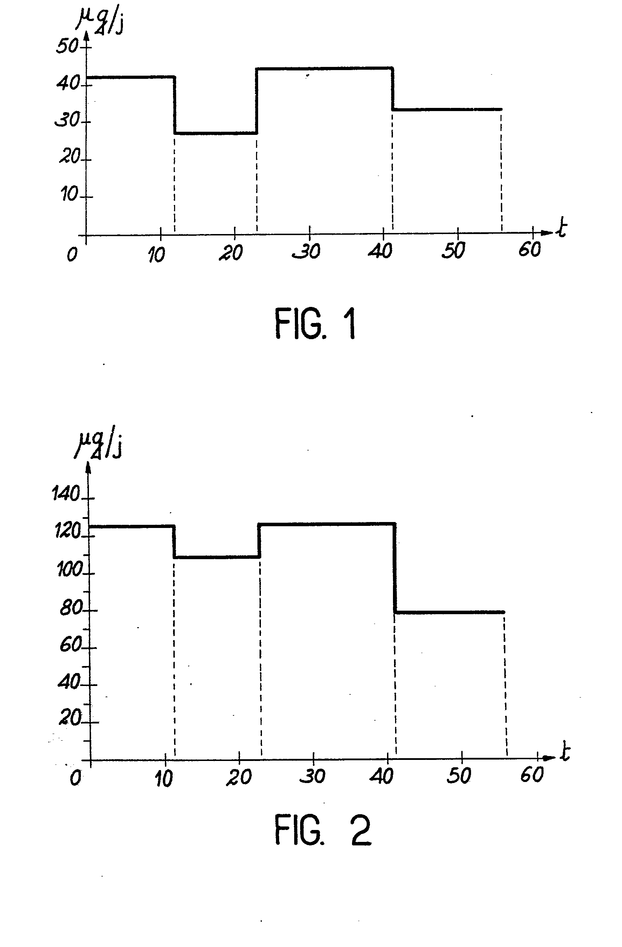

Les résultats obtenus sont donnés sur les fig. 1 et 2 qui se rapportent respectivement à l'utilisation d'une solution ayant un pH de 6,5 et à l'utilisation d'une solution ayant un pH de 5.

Au vu de ces figures qui sont des représentations graphiques en fonction du temps (en jours) de la quantité de cuivre ($\mu$g) libérée par jour dans la solution, on constate que le substrat obtenu est susceptible de libérer chaque jour des quantités sensiblement constantes de cuivre. Par ailleurs, on note que l'emploi d'une solution plus acide conduit à une augmentation de la quantité de cuivre libérée par jour.

### Exemple 2

On prépare de la même façon que dans l'exemple 1 un substrat hydrophobe chargé de poudre de cuivre, en greffant de l'acide acrylique sur une poudre de copolymère d'éthylène et d'acétate de vinyle (EVA) analogue à celle de l'exemple 1, en utilisant la même solution de greffage et le même débit de dose d'irradiation que dans l'exemple 1, mais en réalisant l'irradiation pendant 43 minutes au lieu de 1 h 30.

La poudre greffée obtenue présente un taux de greffage de 30,4 % en poids d'acide acrylique par rapport au poids de poudre de copolymère EVA. Comme dans l'exemple 1, on mélange ensuite la poudre greffée à 12,5 % en poids de poudre de cuivre et on met le mélange sous la forme d'une plaquette de 1 mm d'épaisseur en utilisant le même mode opératoire que dans l'exemple 1, mais en maintenant les plateaux du moule à une température de 150°C au lieu de 170°C.

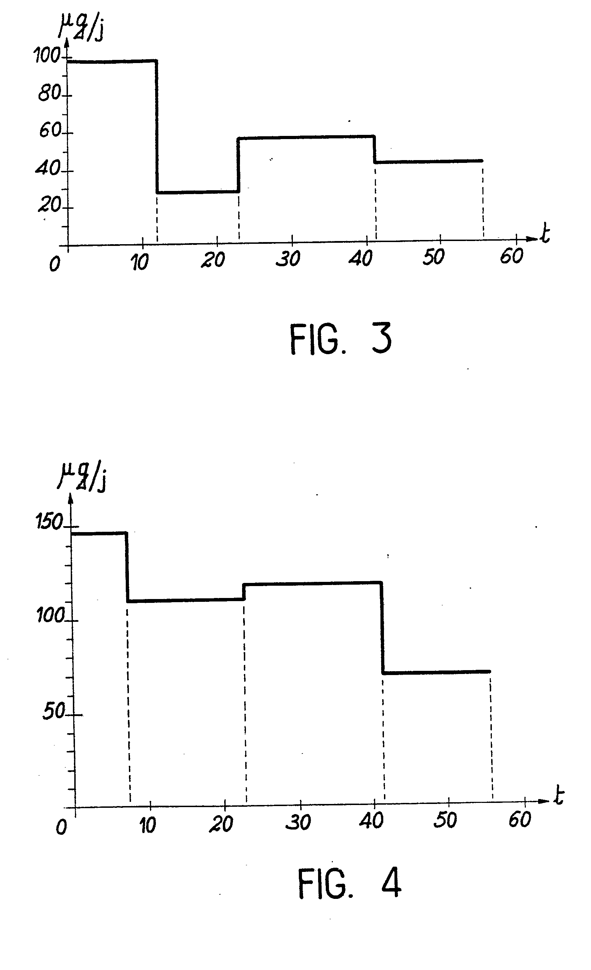

On contrôle ensuite les propriétés de désorption du substrat obtenu sur un morceau de plaquette d'une surface de 600 mm², en utilisant le même mode opératoire que dans l'exemple 1. Les résultats obtenus jusqu'à 56 jours sont donnés sur les figures 3 et 4, qui se rapportent respectivement à l'utilisation d'une solution à pH 6,5 et d'une solution à pH 5, et qui représentent en fonction du temps (en jours) la quantité de cuivre ($\mu$g) libérée par jour dans la solution. Ces résultats confirment également l'influence du pH de la solution sur la quantité de cuivre libérée par le substrat.

### Exemple 3

Cet exemple se rapporte à la réalisation de substrats utilisables comme dispositifs intra-utérins chargés de cuivre.

On greffe tout d'abord de l'acide acrylique sur une poudre de copolymère d'éthylène et d'acétate de vinyle à 33 % en poids d'acétate de vinyle (EVA) ayant une granulométrie moyenne de 0,98 mm.

Dans ce but, on immerge 1 000 g de la poudre de copolymère (EVA) dans la solution de greffage suivante:

— acide acrylique: 500 ml;
— eau permutée: 4 000 ml;
— sel de Mohr: 88 g;

et on la soumet à une irradiation sous rayonnements gamma, au moyen d'une source de cobalt 60, avec un débit de dose d'irradiation de 0,72 Mrad/heure, pendant une durée de 1 h 30, à la température ambiante, en maintenant la solution sous agitation et sous atmosphère d'azote.

Après irradiation, on lave immédiatement la poudre greffée dans de l'eau portée à 90°C, pendant 4 heures, puis on la sèche dans une étuve sous vide jusqu'à poids constant.

On obtient ainsi une poudre greffée dont le taux de greffage est de 31,8 % en poids d'acide acrylique par rapport au poids de la poudre de copolymère EVA.

On mélange ensuite intimement la poudre greffée avec 15 % en poids de poudre de cuivre présentant une granulométrie moyenne de 50 microns et on met ce mélange sous la forme de dispositifs intra-utérins par injection à chaud du mélange dans des moules correspondants à des dispositifs intra-utérins du type OM—GA de taille 0 et de taille 1.

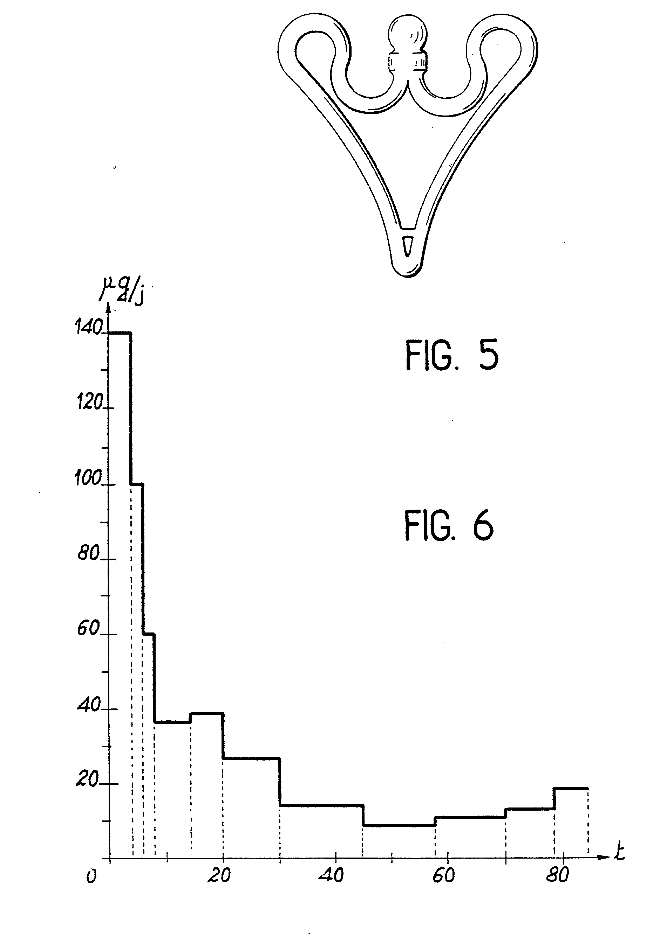

En se reportant à la fig. 5, on voit qu'un dispositif intra-utérin (DIU) de ce type comprend une armature de forme particulière.

Par ailleurs, on précise que le dispositif qui correspond à la taille 0, présente une largeur de 26 mm et une hauteur de 26 mm, et que le dispositif de taille 1 présente une largeur de 35 mm et une hauteur de 35 mm.

On contrôle ensuite les propriétés de désorption des dispositifs intra-utérins ainsi obtenus:

a) soit en plaçant le dispositif intra-utérin dans un appareil en verre d'un volume de 5 $cm^3$ dans lequel circule un débit de liquide isotonique (9 g de NaCl/1, pH 5 ou 6,5) de 400 $cm^3$/jour, l'ensemble de l'appareil étant maintenu à une température de 37°C;

b) soit en plaçant le dispositif intra-utérin dans un récipient contenant un litre de la même solution isotonique, maintenue à une température de 37°C.

Dans les deux cas, on change et on prélève la solution à intervalles réguliers, et on dose la quantité de cuivre présente dans la solution prélevée.

Les résultats obtenus qui sont identiques dans les deux cas, sont donnés sur les fig. 6, 7, 8 et 9 qui représentent graphiquement, en fonction du temps (en jours) a quantité cuivre ($\mu$g) libérée par jour:

a) par le dispositif intra-utérin de taille 0 dans une solution à pH 6,5 (fig. 6);

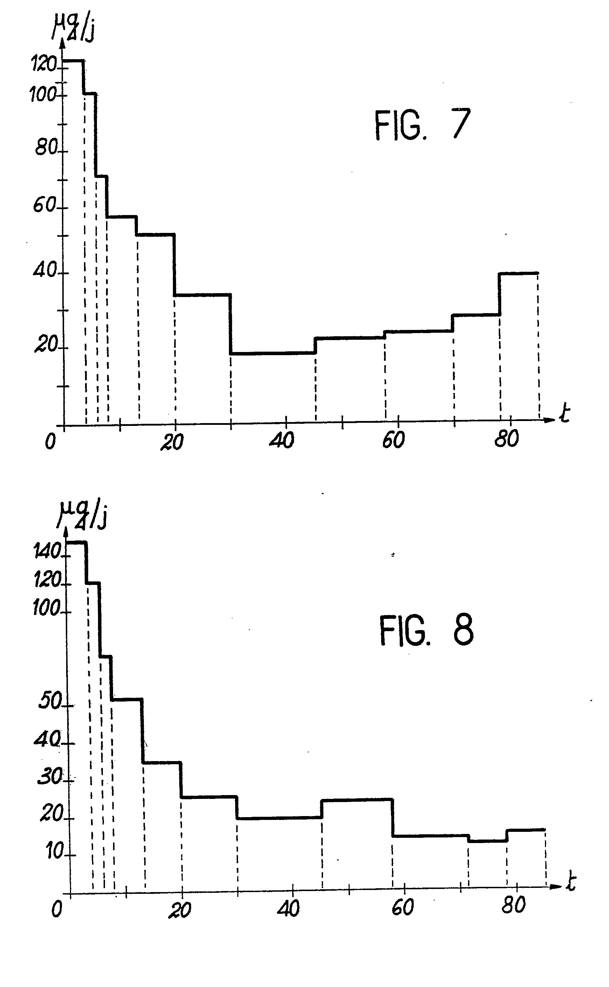

b) le dispositif intra-utérin de taille 0 dans une solution à pH 5 (fig. 7);

c) le dispositif intra-utérin de taille 1 dans une solution à pH 6,5 (fig. 8);

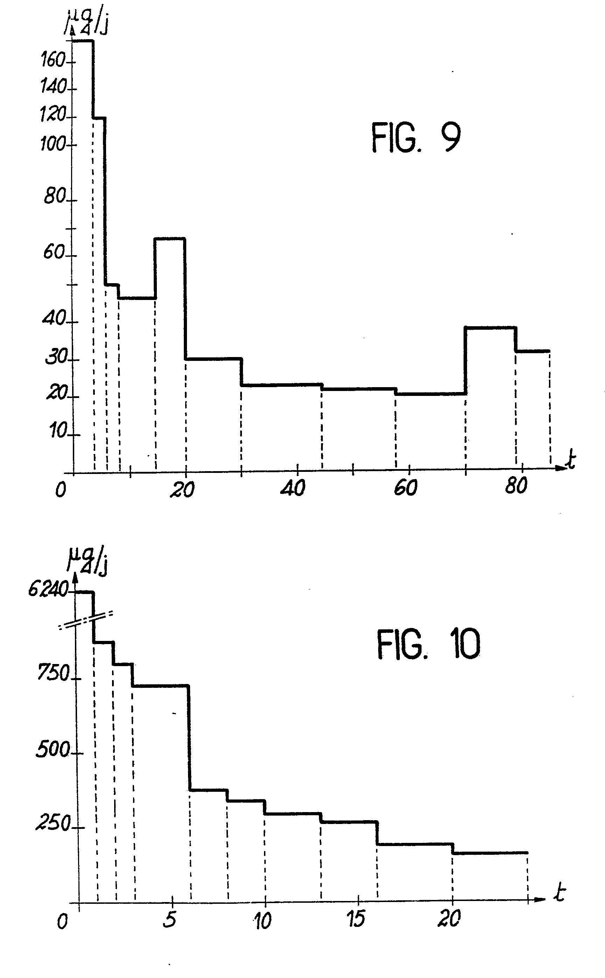

d) le dispositif intra-utérin de taille 1 dans une solution à pH 5 (fig. 9).

On constate au vu de ces figures que la quantité de cuivre libérée par jour se stabilise, après 10 jours, à un taux de 20 à 30 microgrammes par jour et que l'influence de la taille du dispositif intra-utérin et du pH de la solution sont faibles.

Exemple 4

On greffe une poudre de copolymère d'éthylène et d'acétate de vinyle contenant 33 % en poids d'acétate de vinyle (EVA), ayant une granulométrie comprise entre 0,08 et 0,12 mm par de l'acide acrylique en immergeant 625 g de poudre de copolymère EVA dans une solution de greffage contenant:

— 310 $cm^3$ d'acide acrylique;
— 55 g de sel de Mohr;
— 2 500 $cm^3$ d'eau distillée, et

en soumettant la poudre maintenue en agitation dans la solution sous atmosphere d'azote contenant 10 ppm d'oxygène, à une irradiation sous un débit de dose de 0,72 Mrad par heure, pendant une durée de 55 minutes, à la température ambiante.

On lave ensuite la poudre greffée à l'eau et á l'alcool et on la sèche sous vide dans une étuve.

Le taux de greffage de la poudre ainsi obtenue est de 47 % en poids d'acide acrylique par rapport au poids initial de la poudre de copolymère (EVA).

On mélange ensuite 100 g de la poudre greffée ainsi obtenue à 32 g d'acétate de cuivre en poudre, présentant une granulométrie moyenne de 60 microns, puis on met le mélange sous la forme d'une plaque en le comprimant dans un moule, à une température de 150°C.

On contrôle les propriétés de désorption de la plaque obtenue sur un échantillon de 30 x 20 x 2,5 mm prélevé dans la plaque et ayant un poids de 1,751 g. On immerge cet échantillon dans deux litres d'une solution isotonique (9 % de NaCl) dont le pH a été ajusté à 5.

On change à intervalles réguliers la solution de désorption et on détermine la teneur en cuivre de cette solution.

Les résultats obtenus sont représentés sur la fig. 10 qui illustre en fonction du temps (en jours) la quantité de cuivre ($\mu$g) libérée par jour par le substrat.

Exemple 5

On greffe une poudre, de polyamide 11 de granulométrie comprise entre 100 et 125 microns par de l'acrylate de butyle par la technique de préirradiation.

10,74 grammes de poudre polyamide 11 sont irradiés dans une ampoule sous vide sous faisceau d'électrons accélérés de 3 MeV à une dose de 9 Mrad. Toujours sous vide la poudre irradiée est mise en contact avec 100 $m^3$ d'une solution alcoolique d'acrylate de butyle (concentration 1/1), pendant 40 minutes à la température de 70°C.

On lave ensuite la poudre greffée à l'alcool et on la sèche sous vide dans une étuve.

Le taux de greffage de la poudre ainsi obtenue est de 20 % en poids de polyacrylate de butyle par rapport au poids initial de la poudre de polyamide 11.

# 0 009 423

On mélange ensuite 100 grammes de la poudre greffée ainsi obtenue à 20 g de principe actif, l'acide (benzoyl-3 phenyl) 2 propionique (= Ketoprofene) puis on met le mélange sous forme de plaque en le comprimant dans un moule à la température de 90°C.

On détermine la vitesse de désorption du principe actif sur un échantillon de 30 × 20 × 2,5 mm prélevé dans une plaque.

On immerge cet échantillon dans un litre d'alcool éthylique TBG.

On détermine la concentration de l'alcool éthylique en acide (benzoyl-3 phényl) 2 propionique à intervalles réguliers.

La vitesse de désorption reste constante pendant les huit premières heures.

## Exemple 6

Dans les conditions décrites dans l'exemple 1, on met en contact 10,16 grammes de poudre polyamide 11 irradiée à une dose de 9 Mrad sous un faisceau d'électrons accélérés de 3 MeV avec 100 cm³ d'une solution d'acrylate de butyle dans l'alcool éthylique (concentration 1/1) pendant une heure à une température de 70°C.

On lave ensuite la poudre greffée à l'alcool et on la sèche sous vide dans une étuve. Le taux de greffage de la poudre ainsi obtenue est de 40 % en poids de polyacrylate de butyle par rapport au poids initial de la poudre de polyamide 11.

## Exemple 7

Dans les conditions décrites dans l'exemple 1, avec une solution d'acrylate de butyle dans l'alcool éthylique (70/30) 10,10 g de poudre polyamide 11 de granulométrie comprise entre 100 et 125 microns sont greffés à 70°C pendant 30 minutes.

Un taux de greffage de 38 % en poids de polyacrylate de butyle par rapport au poids initial de la poudre polyamide 11 est obtenu.

## Exemple 8

On greffe des granulés de copolymère d'éthylène et d'acétate de vinyle (EVA) contenant 33 % d'acétate de vinyle en utilisant une solution d'acrylate de butyle dans l'alcool éthylique (concentration 25/75).

Pour réaliser le greffage les granulés d'EVA pesés sont introduits dans des ampoules de pyrex contenant la solution d'acrylate de butyle dans l'éthanol à laquelle est ajouté 1 % d'acétate de cuivre et 0,25 % de cuivre en poudre pour inhiber la polymérisation du monomère en solution.

Les ampoules sont dégazées et ensuite irradiées sous rayonnements gamma du cobalt 60 à un débit de dose de 0,2 Mrad.h$^{-1}$ à la température ambiante.

Les résultats suivants ont été obtenus:

| | Solution de greffage | | | |
|---|---|---|---|---|
| Essais | ABu | Ethanol | Dose | % de greffage |
| A | 25 | 75 | 3,4 | 35 |
| B | 35 | 65 | 3,4 | 43 |
| C | 45 | 55 | 3,4 | 66 |

On mélange ensuite 100 g de granulés ainsi greffés à 25 g de principe actif de l'acide (benzoyl-3 phényl) 2 propionique puis on met le mélange sous forme de plaque en le comprimant dans un moule à la température de 80°C.

On détermine la vitesse de désorption comme indiqué dans l'exemple 1.

La vitesse de désorption reste constante pendant les 40 premiers jours.

## Exemple 9

On greffe une poudre de polyéthylène basse densité de granulométrie comprise entre 200 et 400 microns par de l'acrylate d'éthylène glycol par la technique de préirradiation. 10,10 g de poudre sont irradiés dans une ampoule sous vide sous faisceau d'électrons accélérés de 3 MeV à une dose donnée.

Toujours sous vide la poudre irradiée est mise en contact avec 100 cm³ de la solution suivante:

— 50 cm³ d'acrylate d'éthylène glycol;
— 50 cm³ d'eau distillée;
— 1 % de sel de Mohr;

Les résultats suivants ont été obtenus:

7

**0 009 423**

| N° échantillon | Dose | Température | Durée | % de greffage |
|---|---|---|---|---|
| 1 | 6,5 Mrad | 75°C | 1 h | 90 % |
| 2 | 9 Mrad | 75°C | 1 h | 109 % |
| 3 | 9 Mrad | 95°C | 1 h | 75% |

On mélange ensuite 100 g de poudre de polyéthylène greffée dans les conditions de l'échantillon n° 1 avec 30 g de principe actif du phosphate de codéine puis on met le mélange sous forme de plaquette en le comprimant à la température de 40°C.

On détermine la vitesse de désorption du principe actif sur un échantillon de 30 × 20 × 2,5 mm prélevé dans une plaque.

On immerge cet échantillon dans un litre d'eau à pH = 7,4.

On détermine la concentration en phosphate de codéine à intervalles réguliers.

La vitesse de désorption reste constante pendant les huit premières heures.

**Revendications**

1. Substrat hydrophobe apte à libérer au moins une substance chimique, comprenant une matrice de polymère hydrophobe contenant une substance chimique et comportant des inclusions de composés polymérisés, hydrophiles ou hydrophobes, différents du polymère hydrophobe, greffés sur ce polymère hydrophobe, caractérisé en ce que ladite substance est stockée dans la matrice à l'état solide en dehors des inclusions de composés polymérisés.

2. Substrat hydrophobe selon la revendication 1, caractérisé en ce que le polymère hydrophobe est choisi dans le groupe comprenant les acétates de polyvinyle, les polyéthylènes, les polypropylènes, les polyamides, le polytéréphtalate d'éthylène glycol, les chlorures de polyvinyle, les chlorures de polyformaldéhyde, les polycarbonates, les copolymères d'éthylène, les polyéthers, les polyuréthanes et les polyacrilonitriles.

3. Substrat hydrophobe selon la revendication 2, caractérisé en ce que le copolymère d'éthylène est un copolymère d'éthylène et d'acétate de vinyle.

4. Substrat selon l'une quelconque des revendications 1 à 3, caractérisé en ce que les composés polymérisés sont des polymères d'un monomère hydrophile choisi dans le groupe comprenant l'acrylamide, l'acrylate d'éthylène glycol, le méthylol acrylamide, la diacétone acrylamide, l'acide maléique, l'acide acrylique, l'acide fumarique, l'acide itaconique, l'acrylate de propylène glycol, le méthacrylate d'éthylène glycol, la méthacrylamide, l'acide méthacrylique et le méthacrylate de propylène glycol.

5. Substrat selon l'une quelconque des revendications 1 à 3, caractérisé en ce que les composés polymérisés sont des polymères d'un monomère hydrophobe choisi dans le groupe comprenant l'acrylate d'éthyle, l'acrylate de butyle, l'acrylate d'isobutyle, l'acrylate d'hexyle, l'acrylate d'heptyle, le méthacrylate d'éthyle, le méthacrylate de butyle, le méthacrylate d'isobutyle, le méthacrylate d'hexyle, le méthacrylate d'heptyle et l'acrylonitrile.

6. Substrat selon l'une quelconque des revendications 1 à 4, caractérisé en ce que ladite substance chimique est un métal ou un sel métallique.

7. Substrat selon la revendication 6, caractérisé en ce que ladite substance chimique est choisie dans le groupe comprenant le cuivre et l'acétate de cuivre.

8. Substrat selon l'une quelconque des revendications 1 à 3 et 5, caractérisé en ce que ladite substance est l'acide 2-(3-benzoylphényl)-propionique.

9. Substrat selon l'une quelconque des revendications 1 à 4, caractérisé en ce que ladite substance est le phosphate de codéine.

10. Procédé de préparation du substrat hydrophobe selon l'une quelconque des revendications 1 à 9, caractérisé en ce qu'il consiste:

a) à former dans une poudre de polymère hydrophobe des inclusions de composés polymérisés en greffant sur la poudre de polymère hydrophobe un monomère hydrophile ou hydrophobe différent du polymère hydrophobe;

b) à mélanger de façon homogène la poudre greffée ainsi obtenue avec une poudre de ladite substance; et

c) à mettre le mélange sous la forme d'un substrat en le soumettant à un formage à chaud.

11. Procédé selon la revendication 10, caractérisé en ce qu'on réalise le greffage dudit monomère sur ladite poudre de polymère hydrophobe en soumettant à une irradiation au moyen de rayonnements ionisants ladite poudre de polymère hydrophobe immergée dans une solution dudit monomère contenant un inhibiteur de polymérisation.

12. Procédé selon la revendication 11, caractérisé en ce qu'on contrôle le taux de greffage dudit monomère sur le polymère hydrophobe en réglant la température et la concentration en monomère de la solution, le débit de dose d'irradiation et la durée d'irradiation.

13. Procédé selon la revendication 10, caractérisé en ce que la poudre de ladite substance a une granulométrie inférieure à celle de la poudre de polymère hydrophobe greffé.

14. Procédé selon la revendication 10, caractérisé en ce que le formage à chaud est réalisé par injection, par extrusion ou par compression.

15. Procédé selon l'une quelconque des revendications 10 à 14, caractérisé en ce qu'après formage à chaud, on soumet le substrat obtenu à une réticulation au moyen de rayonnements ionisants.

16. Application du substrat hydrophobe selon l'une quelconque des revendications 4, 6 et 7 comme dispositif intra-utérin susceptible de libérer dans l'utérus une substance active.


**Patentansprüche**

1. Zur Abgabe mindestens einer chemischen Substanz fähiges hydrophobes Substrat, enthaltend eine Matrize aus hydrophobem Polymerisat, welche eine chemische Substanz enthält und Einschlüsse von hydrophilen oder hydrophoben polymerisierten Verbindungen, welche vom hydrophoben Polymerisat verschieden sind, auf dieses hydrophobe Polymerisat gepropft enthält, dadurch gekennzeichnet, daß die Substanz in der Matrize in festem Zustand außer den Einschlüssen von polymerisierten Verbindungen gespeichert ist.

2. Hydrophobes Substrat nach Anspruch 1, dadurch gekennzeichnet, daß das hydrophobe Polymerisat ein Polyvinylacetat, ein Polyäthylen, ein Polypropylen, ein Polyamid, Äthylenglycolpolyterephtalat, ein Polyvinylchlorid, ein Polyformaldehydchlorid, ein Polycarbonat, ein Äthylenmischpolymerisat, ein Polyäther, ein Polyurethan und/oder ein Polyacrylnitril ist.

3. Hydrophobes Substrat nach Anspruch 2, dadurch gekennzeichnet, daß das Äthylen-Mischpolymerisat ein Mischpolymerisat von Äthylen und Vinylacetat ist.

4. Hydrophobes Substrat nach einem der Ansprüche 1 bis 3, dadurch gekennzeichnet, daß die polymerisierten Verbindungen Polymerisate eines hydrophilen Monomeren aus der Gruppe Acrylamid, Äthylenglycolacrylat, Methylolacrylamid, Diacetonacrylamid, Maleinsäure, Acrylsäure, Fumarsäure, Itaconsäure, Propylenglycolacrylat, Äthylenglycolmethacrylat, Methacrylamin, Methacrylsäure und Propylenglycolmethacrylat sind.

5. Polymeres Substrat nach einem der Ansprüche 1 bis 3, dadurch gekennzeichnet, daß die polymerisierten Verbindungen Polymerisate eines hydrophoben Monomeren aus der Gruppe Äthylacrylat, Butylacrylat, Isobutylacrylat, Hexylacrylat, Heptylacrylat, Äthylmethacrylat, Butylmethacrylat, Isobutylmethacrylat, Hexylmethacrylat, Heptylmethacrylat und Acrylnitril sind.

6. Hydrophobes Substrat nach einem der Ansprüche 1 bis 4, dadurch gekennzeichnet, daß die chemische Substanz ein Metall oder ein Metallsalz ist.

7. Hydrophobes Substrat nach Anspruch 6, dadurch gekennzeichnet, daß die chemische Substanz Kupfer und/oder Kupferacetat ist.

8. Hydrophobes Substrat nach einem der Ansprüche 1 bis 3 und 5, dadurch gekennzeichnet, daß die Substanz 2-(3-Benzoylphenyl)-propionsäure ist.

9. Hydrophobes Substrat nach einem der Ansprüche 1 bis 4, dadurch gekennzeichnet, daß die Substanz Codeinphosphat ist.

10. Verfahren zur Herstellung eines hydrophoben Substrats nach einem der Ansprüche 1 bis 9, dadurch gekennzeichnet, daß man

a) in einem Pulver eines hydrophoben Polymerisats Einschlüsse von polymerisierten Verbindungen bildet, indem man auf das Pulver des hydrophoben Polymerisats ein hydrophiles oder hydrophobes Monomeres, das von dem hydrophoben Polymerisat verschieden ist, aufpfropft;

b) das so erhaltene gepfropfte Pulver mit einem Pulver der genannten Substanz homogen mischt; und

c) dem Gemisch die Form eines Substrats gibt, indem man es warmverformt.

11. Verfahren nach Anspruch 10, dadurch gekennzeichnet, daß man das Pfropfen des Monomeren auf das Pulver des hydrophoben Polymerisats dadurch bewirkt, indem man das Pulver des hydrophoben Polymerisats, das in eine Lösung des Monomeren, welche einen Polymerisationsinhibitor enthält, eingetaucht ist, mittels ionisierender Strahlungen bestrahlt.

12. Verfahren nach Anspruch 11, dadurch gekennzeichnet, daß man den Pfropfungsgrad des Monomeren auf dem hydrophoben Polymeren dadurch kontrolliert, indem man die Temperatur und die Konzentration der Lösung an Monomeren, die Menge der Strahlungsdosis und die Bestrahlungsdauer regelt.

13. Verfahren nach Anspruch 10, dadurch gekennzeichnet, daß das Pulver der Substanz feinteiliger als das Pulver des gepfropften hydrophoben Polymeren ist.

14. Verfahren nach Anspruch 10, dadurch gekennzeichnet, daß die Warmverformung durch Spritzen, durch Extrudieren oder durch Zusammenpressen erfolgt.

15. Verfahren nach einem der Ansprüche 10 bis 14, dadurch gekennzeichnet, daß man nach der Warmverformung das erhaltene Substrat mit Hilfe ionisierender Bestrahlungen vernetzt.

9

**0 009 423**

16. Verwendung der hydrophoben Substrats nach einem der Ansprüche 4, 6 und 7 als Intrauterin-Pessar, das im Uterus eine aktive Substanz freisetzt.

**Claims**

1. A hydrophobic substrate capable of liberating at least one chemical compound, comprising a hydrophobic polymer matrix containing a chemical compound and comprising inclusions of polymerised compounds, hydrophilic or hydrophobic, different from the hydrophobic polymer and grafted on said hydrophobic polymer, characterized in that said chemical compound is stored in the solid state in the matrix on the outside of the inclusions of polymerized compounds.

2. A hydrophobic substrate according to claim 1, characterized in that the hydrophobic polymer is chosen from the group comprising polyvinyl acetates, polyethylenes, polypropylenes, polyamides, ethylene glycol polyterephtalate, polyvinyl chlorides, polyformaldehyde chlorides, polycarbonates, ethylene copolymers, polyethers, polyurethanes and polyacrylonitriles.

3. A hydrophobic substrate according to claim 2, characterized in that the ethylene copolymer is a copolymer of ethylene and vinyl acetate.

4. A substrate according to any one of claims 1 to 3, characterized in that the polymerized compounds are polymers of a hydrophilic monomer chosen from the group comprising acrylamide, ethylene glycol acrylate, methylol acrylamide, diacetone acrylamide, maleic acid, acrylic acid, fumaric acid, itaconic acid, propylene glycol acrylate, ethylene glycol methacrylate, methacrylamide, methacrylic acid and propylene glycol methacrylate.

5. A substrate according to any one of claims 1 to 3, characterized in that the polymerized compounds are polymers of a hydrophobic monomer chosen from the group comprising ethyl acrylate, butyl acrylate, isobutyl acrylate, hexyl acrylate, heptyl acrylate, ethyl methacrylate, butyl methacrylate, isobutyl methacrylate, hexyl methacrylate, heptyl methacrylate and acrylonitrile.

6. A substrate according to any one of the claims 1 to 4, characterized in that the chemical compound is a metal or a metal salt.

7. A substrate according to claim 6, characterized in that the chemical compound is chosen from the group comprising copper and copper acetate.

8. A substrate according to any one of claims 1 to 3 and 5, characterized in that said chemical compound is 2-(3-benzoylphenyl)-propionic acid.

9. A substrate according to any one of claims 1 to 4, characterized in that said chemical compound is codeine phosphate.

10. A process for the preparation of a hydrophobic substrate according to any one of claims 1 to 9, characterized in that it comprises:

a) forming inclusions of polymerized compounds in a hydrophobic polymer powder by grafting onto the latter a hydrophilic or hydrophobic monomer different from said hydrophobic polymer;

b) homogeneously mixing the grafted powder obtained in this way with a powder of the said chemical compound; and

c) bringing the mixture into the form of a substrate by hot shaping it.

11. A process according to claim 10, characterized in that the monomer is grafted onto the hydrophobic polymer powder by subjecting said powder submerged in a solution of the monomer containing a polymerization inhibitor to irradiation by means of ionizing rays.

12. A process according to claim 11, characterized in that the degree of grafting of the monomer on the hydrophobic polymer is checked by regulating the temperature and monomer concentration of the solution, the irradiation dosage rate and the irradiation duration.

13. A process according to claim 10, characterized in that the powder of the chemical compound has a grain size below that of the powder of the grafted hydrophobic polymer.

14. A process according to claim 10, characterized in that hot shaping is performed by injection moulding, extrusion or compression moulding.

15. A process according to any one of the claims 10 to 14, characterized in that after hot shaping, the substrate obtained undergoes cross-linking by means of ionizing rays.

16. Use of the hydrophobic substrate according to any one of the claims 4, 6 and 7 as an intrauterine device which can liberate an active substance into the uterus.

FIG. 1

FIG. 2

FIG. 3

FIG. 4

FIG. 5

FIG. 6

$\mu g/j$

140

120

100

80

60

40

20

0   20   40   60   80   $t$

FIG. 7

FIG. 8

FIG. 9

FIG. 10